# EUROPEAN PATENT APPLICATION

(11) **EP 2 324 783 A1**
(43) Date of publication of application: **25.05.2011**
(21) Application number: 10251985.7
(22) Date of filing: 23.11.2010
(51) Int. Cl.: A61B 17/34

(54) **Seal anchor introducer including biasing member**

(30) Priority: 24.11.2009 US 236906 P; 03.11.2010 US 938691
(71) Applicant: Tyco Healthcare Group LP, New Haven, CT 06511 (US)
(72) Inventor: Stopek, Joshua, Guilford, CT 06437 (US)
(74) Representative: Soames, Candida Jane

(57) **Abstract**

A seal anchor member has opposing ends that define a longitudinal axis. The seal anchor member is adapted to transition between an expanded condition and a compressed condition to facilitate securing of the seal anchor member within a tissue tract in a substantially sealed relationship. The seal anchor member includes a biasing member positioned along the longitudinal axis. The biasing member is configured and adapted to facilitate the transition between the expanded and compressed conditions. The seal anchor member further includes at least one lumen extending through the seal anchor member for slidably receiving a surgical instrument.

## Description

### CROSS REFERENCE TO RELATED APPLICATIONS

This application claims priority to, and benefit of, U.S. Provisional Application Serial No. 61/263,906, filed November 24, 2009, the disclosure of which is hereby incorporated by reference in its entirety.

### BACKGROUND

### 1. Technical Field

The present disclosure relates to a seal for use in a surgical procedure. More particularly, the present disclosure relates to a seal anchor member adapted for insertion into an incision in tissue, and, for the sealed reception of one or more surgical objects such that a substantially fluid-tight seal is formed with both the tissue and the surgical object, or objects.

### 2. Background of Related Art

Today, many surgical procedures are performed through small incisions in the skin, as compared to the larger incisions typically required in traditional procedures. Generally, such procedures are referred to as "endoscopic", unless performed on the patient's abdomen, in which case the procedure is referred to as "laparoscopic". Throughout the present disclosure, the term "minimally invasive" should be understood to encompass both endoscopic and laparoscopic procedures.

During a typical minimally invasive procedure, surgical objects, such as surgical access devices (e.g., trocar and cannula assemblies) or endoscopes, are inserted into the patient's body through an incision in tissue or into a naturally occurring orifice (e.g., mouth, anus, or vagina). In general, prior to the introduction of the surgical object into the patient's body, insufflation gases are used to enlarge the area surrounding the target surgical site to create a larger, more accessible work area. Accordingly, the maintenance of a substantially fluid-tight seal is desirable so as to prevent the escape of the insufflation gases and the deflation or collapse of the enlarged surgical site.

To this end, various valves and seals are used during the course of minimally invasive procedures and are widely known in the art. However, a continuing need exists for a seal anchor member that can be inserted directly into the incision in tissue and that can accommodate a variety of surgical objects while maintaining the integrity of an insufflated workspace.

### SUMMARY

Disclosed herein is a seal anchor member that is adapted and configured to transition between a first state defining a first length and a second state defining a second length. In particular, the seal anchor member includes a leading portion and a trailing portion. Disposed between the leading and trailing portions is an intermediate portion that is transitionable between the first and second states. A biasing member is disposed in the intermediate portion for transitioning the intermediate portion between the first and second states. The biasing member may be a spring. The biasing member may be biased towards an expanded state. For example, subsequent to compressing the seal anchor member along the longitudinal axis, the seal anchor member may begin to transition back toward an expanded state.

The seal anchor member may include at least one longitudinally extending lumen that extends through the leading and trailing portions of the seal anchor member. The at least one lumen may be configured to receive therein an object in a substantially sealed relation. In an embodiment, the seal anchor member may include one or more lumens that are coaxial with the central longitudinal axis of the seal anchor or are parallel to the longitudinal axis of the seal anchor. The biasing member may be disposed longitudinally about the one or more lumens. In an embodiment, the biasing member may be disposed on or within an outer surface of the intermediate portion.

An example of a seal anchor member may be found in U.S. Pat. Pub. 2009/0093752, the entire contents of which are incorporated herein by reference. The various aspects of the present disclosure will be more readily understood from the following detailed description when read in conjunction with the appended figures.

### BRIEF DESCRIPTION OF THE DRAWINGS

Various embodiments of the present disclosure are described hereinbelow with reference to the drawings, wherein:

Fig. 1 is a front perspective view of a seal anchor member in accordance with the principles of the present disclosure shown in an expanded condition illustrating a seal anchor member positioned relative to body tissue;

Fig. 2 is a front perspective view of the seal anchor member of Fig. 1 shown in a first state;

Fig. 2A is a front perspective view of the seal anchor member of Fig. 1 shown in a second state;

Fig. 3 is a top perspective view of an embodiment of a seal anchor member; and

Fig. 3A is a cross-sectional view of the seal anchor member taken along section line 3A-3A of Fig. 3.

### DETAILED DESCRIPTION OF THE EMBODIMENTS

In the drawings and in the description which follows, in which like reference numerals identify similar or identical elements, the term "proximal" will refer to the end of the apparatus that is closest to the clinician during use, while the term "distal" will refer to the end that is farthest from the clinician during use, as is traditional and known in the art.

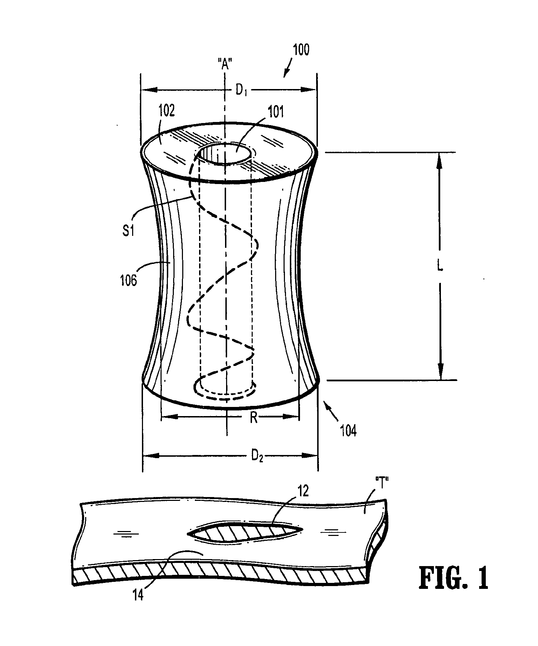

With reference to Figs. 1-2A, a seal anchor member 100 for use in minimally invasive surgical procedures will now be described. The seal anchor member 100 is configured and adapted to be inserted within tissue tract 12 defined by tissue surfaces 14 formed in tissue "T", *e.g*., an incision or a naturally occurring orifice (*e.g*., mouth, anus, or vagina). Seal anchor member 100 defines a longitudinal axis "A" and has respective proximal and distal ends 102, 104 and an intermediate portion 106 disposed between the proximal and distal ends 102, 104.

As depicted in Figs. 1-2A, proximal and distal ends 102, 104 define substantially planar surfaces. However, embodiments are also contemplated herein in which either or both of proximal and distal ends 102, 104 define surfaces that are substantially concave or convex to assist in the insertion of seal anchor member 100 within tissue tract 12. Intermediate portion 106 defines a radial dimension "R" and extends longitudinally between the proximal and distal ends 102, 104 to define an axial dimension or length "L". As shown in Figs. 1-2A, the radial dimension "R" of intermediate portion 106 varies along the length "L", i.e., the cross-sectional dimension may vary long length "L", to facilitate anchoring of the seal anchor member 100 within tissue "T". However, in an embodiment of the seal anchor member 100, the radial dimension "R" may remain substantially uniform along the length "L".

The radial dimension "R" of intermediate portion 106 is appreciably less than the respective diameters D1, D2 of proximal and distal ends 102, 104 such that seal anchor member 100 defines an "hour-glass" shape or configuration to assist in anchoring seal anchor member 100 within tissue "T". However, in an alternate embodiment, the radial dimension "R" of intermediate portion 106 may be substantially equivalent to the respective diameters D1, D2 of proximal and distal ends 102, 104. In cross-section, intermediate portion 106 may exhibit any suitable configuration, e.g., substantially circular, oval, or oblong.

Seal anchor member 100 includes a port 101 disposed about a central longitudinal axis "A" and will be described in more detail below. It is to be understood that in alternative embodiments, port 101 may be positioned differently, e.g., not coaxial about central longitudinal axis "A" and the seal anchor member 100 may include a plurality of ports. In addition, the seal anchor 100 may be devoid of any port.

Port 101 in the absence of a surgical object inserted therein is configured and adapted to prevent the escape of insufflation gas through the port 101. For example, the port 101 1 may be a slit extending the longitudinal length of the seal anchor member 100 through the proximal and distal ends 102, 104. Alternatively, port 101 may define an opening within the seal anchor member 100 having an initial open state having a first inner dimension and upon the introduction of a surgical object, the port 101 transitions to a second state having a second inner dimension configured and adapted to accommodate the surgical object such that a substantially fluid-tight seal is formed therewith by substantially approximating the size and shape of the surgical object to inhibit the escape of insufflation gas through the port 101 in the presence of the surgical object.

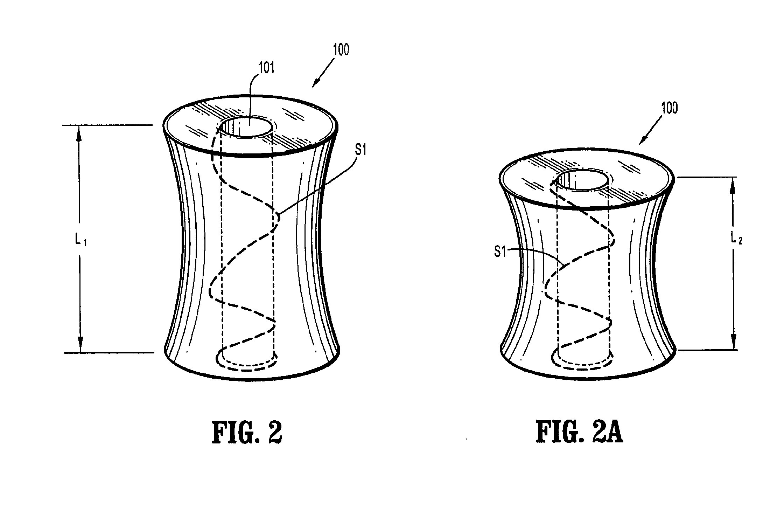

The seal anchor member 100 is configured and adapted to transition between a first state (Fig. 2) and a second state (Fig. 2A) to facilitate insertion of the seal anchor member 100 within tissue "T". As shown in Figs. 1-2A, the seal anchor member 100 includes a biasing member "S1", *e.g*., a spring, that facilitates the transition between a first expanded state shown in Fig. 2 in which the seal anchor member 100 has a length L 1 and a second collapsed state shown in Fig. 2A in which the seal anchor member 100 has a length L2. The biasing member "S1" is biased towards the expanded state as shown in Fig. 2. A surgeon may collapse the seal anchor member 100 such that the seal anchor member 100 has a collapsed length L2. Subsequent to insertion of the seal anchor member 100, biasing member S 1 will facilitate transition back to the expanded condition in which the seal anchor member has a length L1. The seal anchor member 100 will therefore transition toward the expanded condition. The biasing member "S1" is positioned within the seal anchor member 100 such that the biasing member "S1" does not interfere with the function of port 101. It is noted that in other embodiments, the seal anchor member 100 may be biased toward a collapsed state.

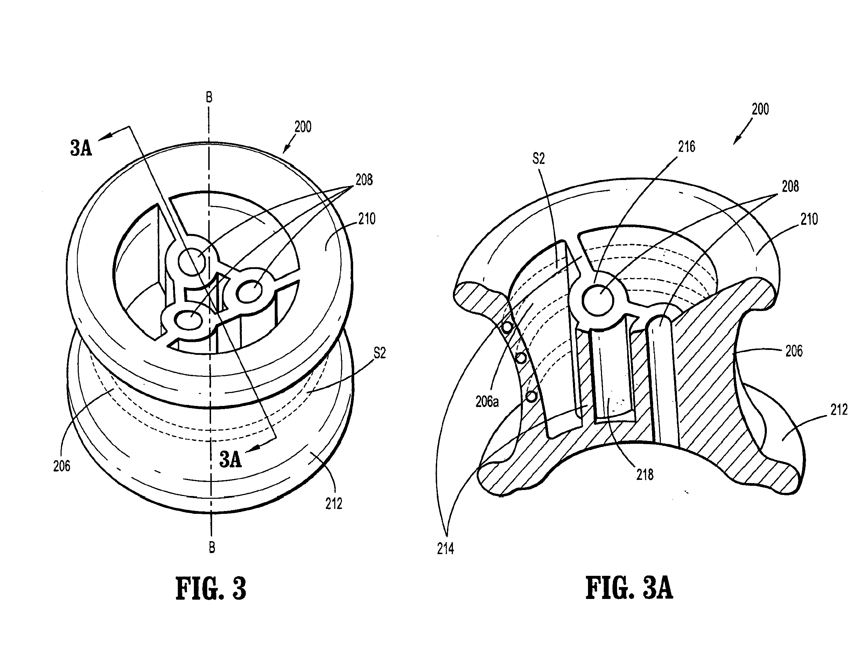

In an alternative embodiment shown in Figs. 3-3A, a seal anchor member 200 having an intermediate portion 206 is shown. A biasing member "S2" may be disposed on or within a surface 206a, i.e., the outer wall, of an intermediate portion 206 extending between proximal rim (trailing end) 210 and distal rim (leading end) 212. Biasing member "S2" is configured and adapted to be biased in a direction along longitudinal axis "B" to facilitate a transition between an expanded first state and a collapsed second state. The seal anchor member 200 may include a plurality of ports 208 secured to the intermediate portion 206 by connective members 214 such that the longitudinal portion of the ports 208 remain substantially constant with respect to the respective proximal and distal rims 210, 212 during insertion and removal of the surgical object.

It is contemplated that biasing members S1, S2 may be formed from any material and have any configuration such that biasing members S1, S2 will have an internal biasing force. For example, biasing members S1, S2 may be a coiled wire and may be formed from a shape memory material, e.g., nitinol, such that biasing members S1, S2 provide a bias in a particular direction to facilitate either the compression or expansion of seal anchor members 100, 200.

Although the illustrative embodiments of the present disclosure have been described herein with reference to the accompanying drawings, the above description, disclosure, and figures should not be construed as limiting, but merely as exemplifications of particular embodiments. It is to be understood, therefore, that the disclosure is not limited to those precise embodiments, and that various other changes and modifications may be effected therein by one skilled in the art without departing from the scope or spirit of the disclosure.

The invention may be described by reference to the following numbered paragraphs:-

A seal anchor member comprising a leading portion; a trailing portion; an intermediate portion disposed between the leading and trailing portions, the intermediate portion transitionable between a first state defining a first length and a second state defining a second length; and a biasing member disposed in the intermediate portion for transitioning the intermediate portion between the first and second states.

The seal anchor of paragraph [0026], wherein the biasing member is a spring.

The seal anchor of paragraph [0026], wherein the biasing member is biased towards an expanded state.

The seal anchor of paragraph [0026] further comprising at least one lumen longitudinally extending through the leading and trailing portions.

The seal anchor of paragraph [0029], wherein the at least one lumen is configured to receive an object therein a substantially sealed relation.

The seal anchor of paragraph [0029], wherein the at least one lumen is coaxial with a central longitudinal axis of the intermediate portion.

The seal anchor of paragraph [0026], wherein the biasing member is disposed on or within an outer surface of the intermediate portion.

## Claims

1. A seal anchor member comprising:
a leading portion;
a trailing portion;
an intermediate portion disposed between the leading and trailing portions, the intermediate portion transitionable between a first state defining a first length and a second state defining a second length; and
a biasing member disposed in the intermediate portion for transitioning the intermediate portion between the first and second states.

2. The seal anchor of claim 1, wherein the biasing member is a spring.

3. The seal anchor of claim 1 or claim 2, wherein the biasing member is biased towards an expanded state.

4. The seal anchor of any preceding claim further comprising at least one lumen longitudinally extending through the leading and trailing portions.

5. The seal anchor of claim 4, wherein the at least one lumen is configured to receive an object therein a substantially sealed relation.

6. The seal anchor of claim 4 or claim 5, wherein the at least one lumen is coaxial with a central longitudinal axis of the intermediate portion.

7. The seal anchor of any preceding claim, wherein the biasing member is disposed on or within an outer surface of the intermediate portion.
